(19) Europäisches Patentamt
European Patent Office
Office européen des brevets

(11) **EP 2 579 035 A1**

(12) **EUROPEAN PATENT APPLICATION**
published in accordance with Art. 153(4) EPC

(43) Date of publication:
**10.04.2013 Bulletin 2013/15**

(21) Application number: **11789647.2**

(22) Date of filing: **23.05.2011**

(51) Int Cl.:
*G01N 33/493* (2006.01)     *G01N 21/78* (2006.01)
*G01N 33/483* (2006.01)

(86) International application number:
**PCT/JP2011/061753**

(87) International publication number:
**WO 2011/152238 (08.12.2011 Gazette 2011/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2010 JP 2010126719**

(71) Applicant: **Arkray, Inc.**
**Minami-ku
Kyoto-shi
Kyoto 601-8045 (JP)**

(72) Inventors:
• **NAKAJIMA Shinya**
**Kyoto-shi
Kyoto 602-0008 (JP)**
• **KUBO Kosuke**
**Kyoto-shi
Kyoto 602-0008 (JP)**

(74) Representative: **Piésold, Alexander James**
**Dehns
St Bride's House
10 Salisbury Square
London
EC4Y 8JD (GB)**

(54) **URINE ANALYSIS METHOD, DEVICE THEREOF, PROGRAM USED IN URINE ANALYSIS METHOD, AND STORAGE MEDIUM THEREOF**

(57) An analysis device AN includes determination means 6, capable of performing a first determination on whether a specific component in urine is positive or negative, on the basis of a color reaction between a reagent and the specific component in urine; and optical measurement means 7B capable of working out data on light absorption characteristics of the urine itself with respect to light of a predefined wavelength region. When the data on the light absorption characteristics lies within a predefined range, and a result of the first determination is either positive or negative as established beforehand, the determination means 6 changes the result of the first determination to a false positive or a false negative, or performs second determination to the effect that there is a likelihood of a false positive or a false negative. As a result, it becomes possible to determine accurately whether a component in urine, if any, yields a false positive or false negative, or to determine that there is a likelihood of such an occurrence. A highly reliable testing result can be achieved thereby.

FIG.2

**Description**

TECHNICAL FIELD

The present invention relates to a technology that can be used in analysis of urine using reagents in, for example, urine testing in medical examinations.

BACKGROUND ART

Conventional examples of urine analysis methods include, for instance, the method disclosed in Patent document 1. In the analysis method disclosed in Patent document 1, the concentration of a specific component in urine is determined using test paper that is coated with a reagent. The reagent develops color by reacting with the specific component in the urine, such that the degree of resulting coloration corresponds to the concentration of the specific component. The coloration degree of the reagent is obtained, in the form of an optical numerical value, using an optical instrument. The concentration of the specific component in urine can be then worked out on the basis of that numerical value. In this analysis method, the color tone of urine itself is tested, using the optical instrument, in a state where the urine is not in contact with the reagent. If the urine has color, the coloration state of the reagent may in some instances be affected by the color of the urine. By testing the color of the urine, it becomes accordingly possible to correct the value of the concentration of the specific component when working the concentration out on the basis of the coloration degree of the reagent.

However, the above-described conventional technology has the following drawbacks.

In a case where the urine analysis process is performed using a reagent, the analysis result may in some instances not correspond to actual values, due to the influence of a drug, if any, that is being administered to the subject. A case of testing of bilirubin in urine qualitative testing will be explained next as an example of such an occurrence. The azo coupling method is widely used in bilirubin testing. In the azo coupling method, a diazo reagent reacts with bilirubin under acidic conditions (diazo coupling reaction), and an azo dye is formed as a result. However, reactions in which an azo dye is thus formed are not specific to bilirubin, and may take place if the subject is being administered drugs such as iobenzamic acid, Etodolac, Epalrestat or the like. Therefore, instances, where the reaction of the reagent yields a positive of bilirubin, may actually be instances of false positive. In the above-described conventional technology it is difficult to discriminate accurately between whether the result is actually a false positive or a false negative. The reliability of the testing result was therefore a shortcoming.

Patent document 1: Japanese Patent Application Publication No. H7-35744

DISCLOSURE OF THE INVENTION

It is an problem of the present invention, which was arrived at in the light of the above considerations, to provide a urine analysis method, an analysis device, a program that is used in the urine analysis method, and a storage medium of that program, that allow accurately determining whether a component in urine yields a false positive or a false negative, or that there is a likelihood of such an occurrence, and that allow obtaining highly reliable testing results.

In order to solve the above problem, the present invention relies on the following technical means.

A urine analysis method provided in a first aspect of the present invention includes: a first determination step of, on the basis of a color reaction between a reagent and a specific component in urine, determining a distinction between a positivity and negativity of the specific component; and a step of working out data on light absorption characteristics of the urine itself with respect to light of a predefined wavelength region, this method further including: a second determination step of, when a value of the data on the light absorption characteristics lies within a predefined range and a determination result in the first determination step is either positive or negative as established beforehand, changing the determination result to a false positive or false negative, or determining that there is a likelihood of a false positive or a false negative.

In the second determination step, preferably, a value of the data on the light absorption characteristics is determined to lie within the predefined range when the urine is of a predefined colorless grade or yellow grade.

Preferably, the specific component in the urine is bilirubin, and when the value of the data on the light absorption characteristics lies within the predefined range and the determination in the first determination step has turned out to be bilirubin positive, in the second determination step the positive is changed to a false positive or determination is made that there is a likelihood of a false positive.

In the urine analysis method according to the present invention, preferably, the data on the light absorption characteristics is data denoting light absorption characteristics of the urine with regard to light of a wavelength in the vicinity of 525 nm and of a wavelength in the vicinity of 470 nm.

In the urine analysis method according to the present invention, preferably, it is determined, in the second determination step, that the value of the data on the light absorption characteristics lies within the predefined range when there hold both relationships in expressions A1<0.08, A2<0.5 , where A1 denotes absorbance of the urine with respect to light of wavelength in the vicinity of 525 nm, as absorbance per 10 mm of optical path length, and A2 denotes absorbance of the urine with respect to light of wavelength in the vicinity of 470 nm as absorbance per 10 mm of optical path length.

In the urine analysis method according to the present invention, preferably, the data on the light absorption characteristics is a calculated value B that is calculated according to expression:

$$B=(A4-A5)/(A3-A5)$$

(where A3, A4 and A5 are, respectively, the absorbance of the urine with regard to light of a wavelength in the vicinity of 525 nm, a wavelength in the vicinity of 470 nm and of a wavelength in the vicinity of 635 nm).

In the urine analysis method according to the present invention, preferably, determination is made as to whether a relationship B<5.30 holds or not.

In the urine analysis method according to the present invention, preferably, when the relationship B<5.30 holds, determination is made, in the second determination step, that the value of the data on the light absorption characteristics lies within the predefined range.

In the urine analysis method according to the present invention, preferably, when the relationship B<5.30 does not hold, determination is further made as to whether a relationship A6<0.54 holds or not, and when the relationship A6<0.54 holds, determination is made, in the second determination step, that the value of the data on the light absorption characteristics lies within the predefined range (where A6 denotes absorbance of the urine with respect to light of wavelength in the vicinity of 470 nm, as absorbance per 10 mm of optical path length).

Preferably, the urine analysis method according to the present invention further includes a step of, when predefined abnormal coloration occurs in the color reaction, detecting that occurrence.

An analysis device provided according to a second aspect of the present invention comprises determination means capable of, on the basis of a color reaction between a reagent and a specific component in urine, performing a first determination of distinguishing between a positivity and negativity of the specific component, and optical measurement means capable of working out data on light absorption characteristics of the urine itself with regard to light of a predefined wavelength region, wherein the determination means is configured so that, when a value of the data on the light absorption characteristics lies within a predefined range and a determination result in the first determination is either positive or negative, as established beforehand, the determination means changes the result of the first determination to a false positive or false negative, or performs a second determination to the effect that there is a likelihood of a false positive or a false negative.

In the second determination in the analysis device according to the present invention, preferably, the value of data on the light absorption characteristics is determined to lie within the predefined range when the urine is of a predefined colorless grade or yellow grade.

In the analysis device according to the present invention, preferably, the determination means is configured so that, in case of determining a distinction between a positivity and negativity of bilirubin as the specific component in the urine, and when the value of the data on the light absorption characteristics lies within the predefined range and a result of the first determination is bilirubin positive, the determination means, in the second determination, changes the positive to a false positive or determines that there is a likelihood of a false positive.

In the analysis device according to the present invention, preferably, the data on the light absorption characteristics is data denoting light absorption characteristics of the urine with regard to light of a wavelength in the vicinity of 525 nm and of a wavelength in the vicinity of 470 nm.

In the analysis device according to the present invention, preferably, determination is made, in the second determination, that the value of data on the light absorption characteristics lies within the predefined range when there hold both relationships in expressions: A1<0.08, A2<0.5, where A1 denotes absorbance of the urine with respect to light of the wavelength in the vicinity of 525 nm and absorbance per 10 mm of optical path length, and A2 denotes absorbance of the urine with respect to light of the wavelength in the vicinity of 470 nm and absorbance per 10 mm of optical path length.

In the analysis device according to the present invention, preferably, the data on the light absorption characteristics is a calculated value B that is calculated according to expression:

$$B=(A4-A5)/(A3-A5)$$

(where A3, A4 and A5 are, respectively, the absorbance of the urine with respect to light of a wavelength in the vicinity of 525 nm, a wavelength in the vicinity of 470 nm and of a wavelength in the vicinity of 635 nm.)

The analysis device according to the present invention, preferably, is configured so as to determine whether B<5.30 holds or not.

In the analysis device according to the present invention, preferably, when the relationship B<5.30 holds, determination is made, in the second determination, that the value of the data on the light absorption characteristics lies within the predefined range.

In the analysis device according to the present invention, preferably, when the relationship B<5.30 does not hold, determination is further made as to whether a relationship A6<0.54 holds or not, and when the relationship A6<0.54 holds, determination is made, in the second determination, that the value of the data on the light absorption characteristics lies within the predefined range (where A6 denotes absorbance of the urine with respect to light of wavelength in the vicinity of 470 nm and absorbance per 10 mm of optical path length).

In the analysis device according to the present invention, preferably, data for identifying the result of the second determination can be outputted using data output means.

The analysis device according to the present invention, preferably, is configured in such a manner that when predefined abnormal coloration occurs in the color reaction, that occurrence is detected.

A program provided by a third aspect of the present invention is a program for causing determination means of an analysis device to perform data processing, the analysis device being provided with: determination means capable of, on the basis of a color reaction between a reagent and a specific component in urine, performing a first determination of distinguishing between a positivity and negativity of the specific component; and optical measurement means capable of working out data on light absorption characteristics of the urine itself with regard to light of a predefined wavelength region, wherein the program comprises data for causing the determination means to execute a second determination of, when a value of the data on the light absorption characteristics lies within a predefined range and a determination result in the first determination step is either positive or negative, as established beforehand, changing the determination result to a false positive or false negative, or determining that there is a likelihood of a false positive or a false negative.

A storage medium provided by a fourth aspect of the present invention is characterized by storing the program provided by the third aspect of the present invention.

Other features and advantages of the present invention will become more apparent from the description of embodiments of the invention set forth below with reference to accompanying drawings.

BRIEF DESCRIPTION OF THE DRAWINGS

Fig. 1 is a schematic perspective-view diagram illustrating an example of an analysis device according to the present invention;
Fig. 2 is a block diagram of the analysis device illustrated in Fig. 1;
Fig. 3 is a schematic explanatory diagram illustrating the configuration of an essential portion of the analysis device illustrated in Fig. 1;
Fig. 4 is an essential-portion perspective-view diagram of an optical measurement unit for color reactions in the analysis device illustrated in Fig. 1;
Fig. 5 is a cross-sectional diagram of Fig. 3 along V-V;
Fig. 6 is a flowchart illustrating an example of an operation processing procedure in a control unit of the analysis device illustrated in Fig. 1;
Fig. 7 is a diagram illustrating an example of a report that is outputted by the analysis device illustrated in Fig. 1;
Fig. 8 is a diagram illustrating another example of a report that is outputted by the analysis device illustrated in Fig. 1;
Fig. 9 is diagram for explaining a relationship between the wavelength regions of light for measuring the absorbance of urine itself and the absorption spectrum of bilirubin itself that is comprised in the urine, in the analysis device illustrated in Fig. 1; and
Fig. 10 is a flowchart illustrating another example of an operation processing procedure in the control unit of the analysis device illustrated in Fig. 1.

BEST MODE FOR CARRYING OUT THE INVENTION

Preferred embodiments of the present invention are explained below with reference to accompanying drawings.

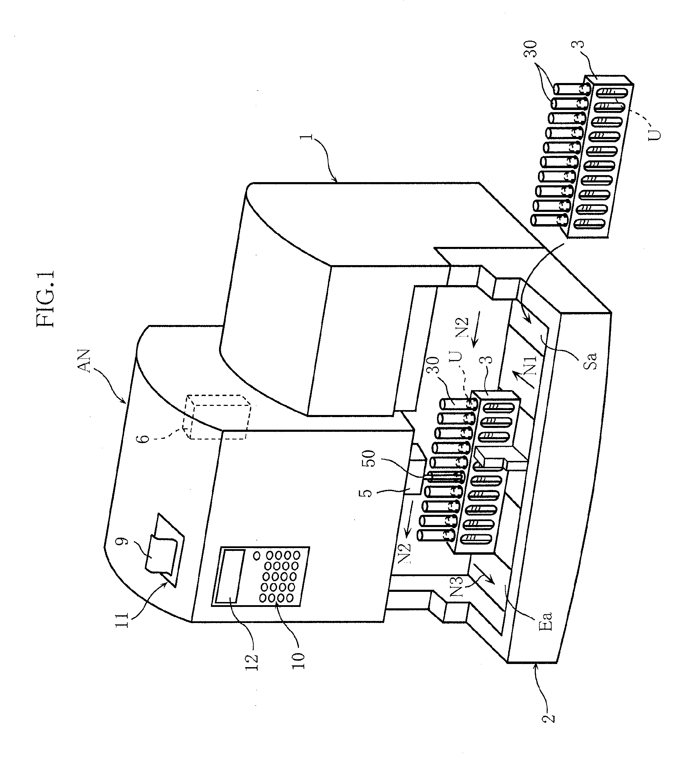

Fig. 1 to Fig. 5 illustrate an example of an analysis device in which the present invention is used. As Fig. 1 shows, an analysis device AN of the present embodiment is a device for performing an analysis process of urine U that is held in containers 30. In the configuration of the analysis device AN, a transport device 2 is assembled with the front face section of an analysis device main body 1.

The transport device 2 is a device for transporting a rack 3, which holds the upright containers 30, along a given pathway. The transport device 2 can be configured in the same way as in known conventional transport devices (for instance, the transport device disclosed in Japanese Patent Application Publication No. 2009-229233), and a detailed explanation of the specific structure involved will be omitted. In the transport device 2, once the rack 3 is brought to a predefined starting-point region Sa, the rack 3 is sequentially transported thereafter along the direction denoted by arrows N1 to N3, to reach ultimately a predefined end-point region Ea. An operation of sampling urine U out of the containers 30 is performed, by a below-described suction nozzle 50, during the process in which the rack 3 is transported in the direction of arrow N2.

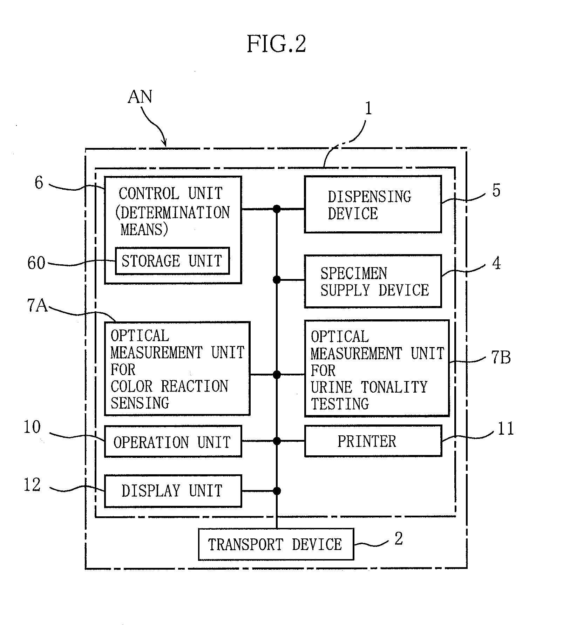

As illustrated in Fig. 2, the analysis device AN comprises, in addition to the abovementioned transport device 2, also a specimen supply device 4, a dispensing device 5, a control unit 6, an optical measurement unit for color reaction sensing 7A, an optical measurement unit for urine tone testing 7B, an operation unit 10, a printer 11 and a display unit 12.

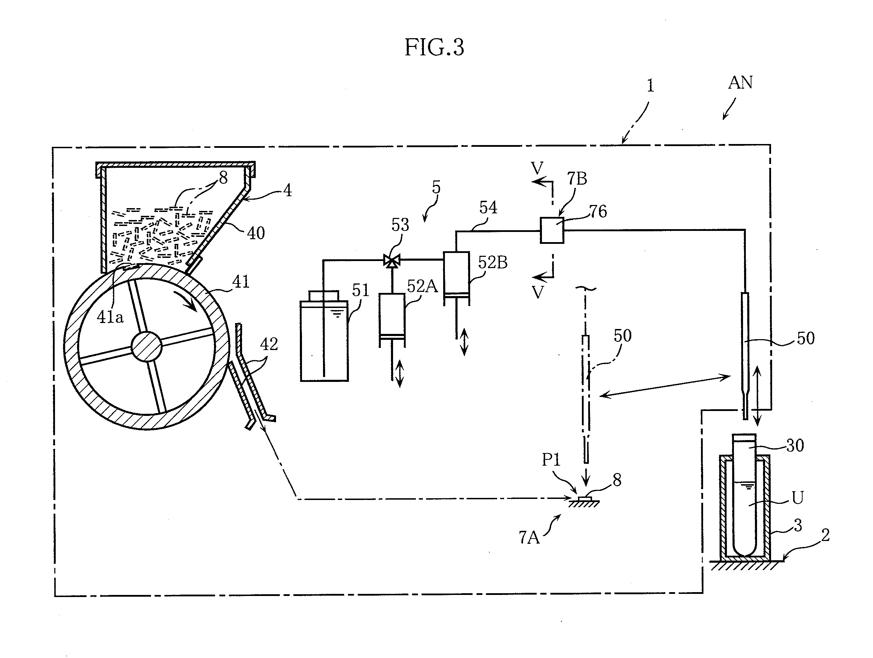

As illustrated in Fig. 3, the specimen supply device 4 is a device for supplying specimens 8 for urine analysis to a predefined site P1 of the optical measurement unit 7A. The specimen supply device 4 comprises a hopper 40 that holds a plurality of specimens 8, and a rotating drum 41 for retrieving the specimens 8, one by one, from the hopper 40. The rotating drum 41 has a recess 41a such that only one specimen 8 can fit into the outer peripheral face thereof. Through rotation of the rotating drum 41, the specimen 8 that is fitted into the recess 41a is transferred out of the hopper 40, into within a pair of guides 42. Thereafter, the specimen 8 is transferred to the predefined site P1 by a transfer device, not shown.

The dispensing device 5 is capable of performing an operation of sampling urine U out of the containers 30, by way of the suction nozzle 50, and dispenses (by spotting) the sampled urine U onto the specimen 8. The suction nozzle 50 can move vertically and horizontally by virtue of a driving mechanism, not shown. The dispensing device 5 has the function of cleaning the suction nozzle 50, and is provided with a cleaning solution tank 51 that stores a cleaning solution such as distilled water, syringe pumps 52A, 52B, a directional control valve 53 such as a three-way valve, and a flow channel 54 serially formed from the cleaning solution tank 51 up to the suction nozzle 50. The flow channel 54 is configured using an appropriate tube. Negative pressure for suction of the urine U, or positive pressure for discharge of the urine U, can be created inside the suction nozzle 50 through operation of the syringe pumps 52A, 52B. Once discharge of the urine U is over, the cleaning solution in the cleaning solution tank 51 is fed into the suction nozzle 50. The latter can be cleaned thereby. Such a configuration is identical, for instance, to that of the dispensing device disclosed in Japanese Patent Application Publication No. 2000-321270, and will not be explained in detail. In the present embodiment, the optical measurement unit for urine tone testing 7B is provided at a site halfway in the flow channel 54 of the dispensing device 5. This feature will be explained further on.

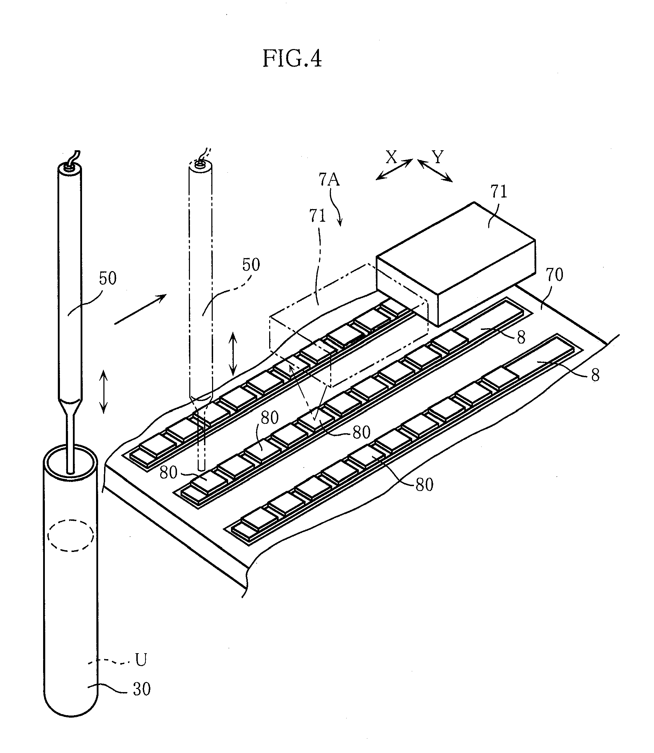

As illustrated in Fig. 4, the optical measurement unit for color reaction sensing 7A comprises a stage 70 for placing the plurality of specimens 8, and an optical measurement device 71. A plurality of reagent pads 80 are provided for each specimen 8. The urine U sampled by the suction nozzle 50 is dispensed onto the reagent pads 80. The plurality of reagent pads 80 comprise reagents that react with predefined components in the urine U, and that develop color in a degree corresponding to the concentration of respective components. The reagents include various components that correspond to the testing items of the urine U. For instance, a diazo reagent is used as a reagent for bilirubin testing, as explained in the section on background art. The optical measurement device 71 can move in the X and Y directions. After spotting of the urine U, light of a predefined wavelength region is irradiated, out of a light source (not shown), onto the reagent pads 80, and the reflected light from the reagent pads is received by a light-receiving element (not shown). The light reflectance of the reagents can be measured on the basis of the amount of received light in the light-receiving element. The light reflectance corresponds to the degree of the color reaction (coloration degree) between the reagent and the specific component of the urine U, such that the presence or absence of the specific component, or the concentration thereof (including semi-quantitative values) in the urine, are determined on the basis of that value.

To work out the abovementioned light reflectance, a so-called two-wavelength method can be adopted that relies on main light and reference light. For instance, the wavelength of the main light is 565 nm or about 565 nm, and the wavelength of the reflectance is 760 nm or about 760 nm. The reference light helps reduce errors that are caused by, for instance, variability in size and other parameters of the specimens 8. Examples of arithmetic expressions for working out the reflectance are explained further on. The optical measurement unit 7A is configured in such a manner that the latter is capable of appropriately detecting abnormal coloration, if any, that may occur after dispensing of the urine U onto the reagent pads 80. To detect abnormal coloration, the wavelength of main light is for instance 565 nm or about 565 nm, as described above, whereas the wavelength of the reference light is 500 nm or about 500 nm. A scheme for determining abnormal coloration is explained further on.

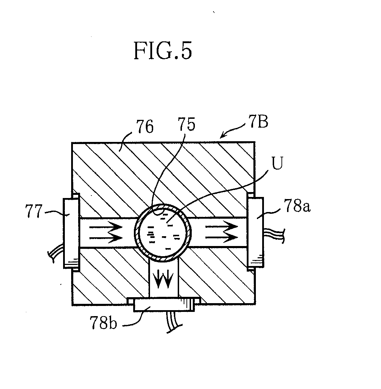

As Fig. 3 shows, the optical measurement unit for urine tone testing 7B is provided at a site halfway in the flow channel 54, such that the urine U that is suctioned by the suction nozzle 50 can flow into a below-described cell 75 of the optical measurement unit 7B. As Fig. 5 shows, the optical measurement unit 7B has a transparent cylindrical cell 75 into which urine U flows, a light source 77 mounted onto a light shielding block 76 that surrounds the cell 75, and two light-receiving elements 78a, 78b. The light source 77 can irradiate light towards the cell 75. As the light there can be used light of a total three wavelengths, for instance 470 nm, 525 nm and 635 nm, or values in the vicinity thereof. The light-receiving element 78a receives light that passes through urine U in the cell 75. Predetermined absorbances of urine U per optical path length A1, A2 and so forth, which will be described below, are worked out on the basis of the amount of received light in the light-receiving element 78a. The absorbances A1, A2 and the like are the absorbance of the urine U itself that does not react with the reagent. The purpose of the light-receiving element 78b is to receive light that is scattered and reflected by the urine U. The turbidity of the urine U can be determined on the basis of the amount of received light in the light-receiving element 78b.

The absorbance of light at a given wavelength (for instance, the abovementioned absorbances A1, A2 and so forth) is worked out from absorbance $=\log_{10}(I_0/I)$, where $I_0$ and I denote, respectively, the intensity of transmitted light of a solvent (for instance, water) and a solution (for instance, urine) of identical optical path length. The intensity of incident light onto the solution may also be used as $I_0$. Absorbance is proportional to the optical path length. Therefore, absorbance measured at a given optical path length can be converted to absorbance at another optical path length, as described below. As the absorbance there is ordinarily used absorbance per 10 mm of optical path length.

In Fig. 2, the purpose of the printer 11 is to print out, in predefined paper, the analysis results of urine U as well data on as other predefined items. The printer 11 corresponds to an example of the data output means in the present invention. The display unit 12 is provided with an image display screen such as a liquid crystal display panel, and performs, for instance, screen display for guiding the operation of the operation unit 10. The analysis result of the urine U may be displayed on the display unit 12. In this case, the display unit 12 corresponds to a specific example of the data output means in the present invention.

The control unit 6 is configured, for instance, using a microcomputer, and comprises a storage unit 60. The control unit 6 corresponds to an example of the determination means in the present invention. In the storage unit 60 there is stored a program and various data items for execution of operation control of the various units of the analysis device AN and execution of various instances of data processing. An example of a specific operation process of the control unit 6 is explained further on.

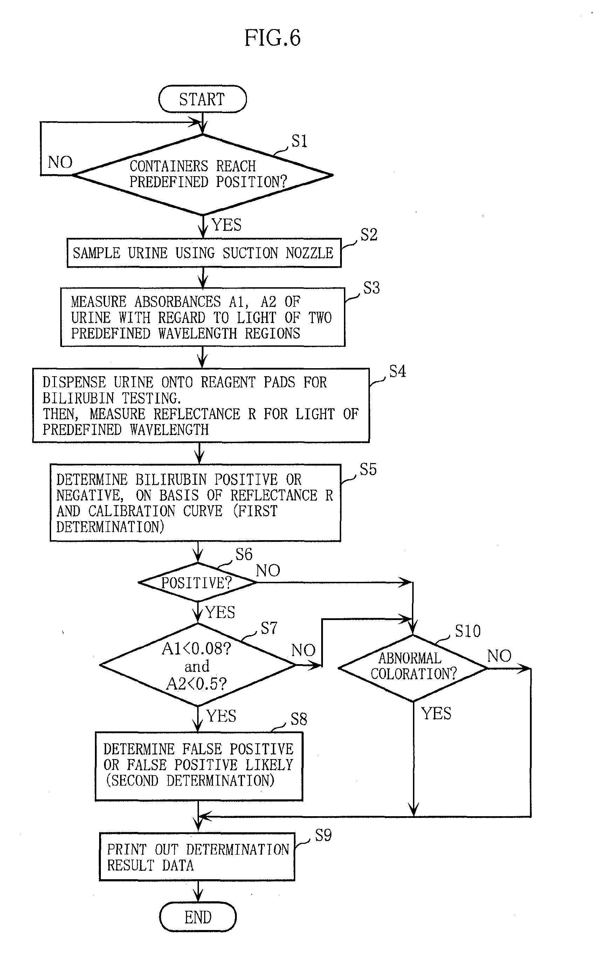

An example of an analysis method of the urine U that utilizes the analysis device AN is explained next. As a typical example of the method an instance will be explained of testing of positive and negative bilirubin in the urine U. An example of the operation processing procedure of the control unit 6 will be explained with reference to the flowchart illustrated in Fig. 6.

Firstly, the transport device 2 is driven, to cause thereby the rack 3 to be transported along the abovementioned given pathway. When the containers 30 reach a predefined position in the course of such transport (S1: YES), the control unit 6 drives the dispensing device 5, and urine U is sampled out of the containers 30 by the suction nozzle 50 (S2). Some of the sampled urine U is infused into the cell 75 of the optical measurement unit for urine tone testing 7B. The control unit 6 causes light to be irradiated, towards the urine U, out of the light source 77 of the optical measurement unit 7B, and the absorbances A1, A2 of the urine U with regard to light of the two wavelength regions is worked out from the amount of transmitted light (S3).

The absorbance A1 is the absorbance of the urine U with regard to light of, for instance, a wavelength of 525 nm or about 525 nm, and denotes absorbance per 10 mm of optical path length. For instance, in a case where the inner diameter (optical path length of the urine region) of the cell 75 illustrated in Fig. 5 is 1.8 mm, the absorbance A1, converted as the absorbance per 10 mm of optical path length, can be worked out by multiplying the value of absorbance at an optical path length of 1.8 mm, obtained using the cell 75, by a value of (10/1.8). The absorbance A2 is the absorbance of the urine U with regard to light of, for instance, a wavelength of 470 nm or about 470 nm, and results from conversion to absorbance per 10 mm of optical path length. The absorbances A1, A2 are used for determining bilirubin false positives, as described below. The absorbance worked out using the cell 75 can be converted to absorbance per other optical path length, for instance, 5 mm, 15 mm and 20 mm.

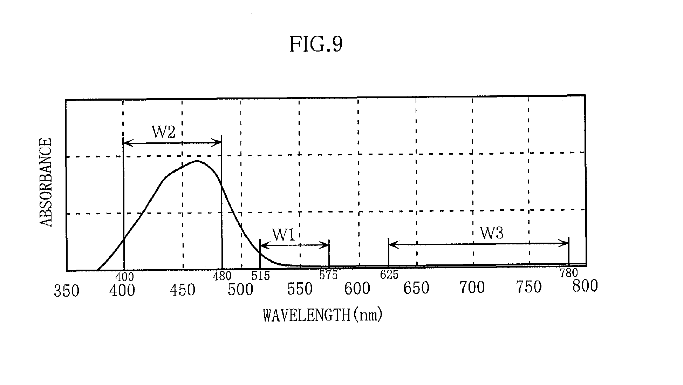

Fig. 9 illustrates the relationship between the absorption spectrum of bilirubin itself comprised in the urine and the measurement wavelength of the absorbances A1, A2. As described above, the measurement wavelength of the absorbance A1 is, for instance, a wavelength of 525 nm or about 525 nm. As Fig. 9 shows, the measurement wavelength of the absorbance A1 matches the wavelength at the foot portion of the absorption of bilirubin. Specifically, the measurement wavelength of the absorbance A1 is preferably selected from 515 nm to 575 nm (wavelength region W1). As pointed above, the measurement wavelength of the absorbance A2 is, for instance, 470 nm or about 470 nm. The measurement wavelength of the absorbance A2 matches the wavelength at a main absorption portion of bilirubin. Specifically, the measurement wavelength of the absorbance A2 is preferably selected from 400 nm to 480 nm (wavelength region W2).

The urine U sampled by the suction nozzle 50 is spotted on the reagent pads 80 for bilirubin testing of the specimens 8. Thereafter, light of two wavelengths is sequentially irradiated onto the spotting portions, to measure thereby the reflectance R of light from the spotting portion (S4). In a case where the urine U comprises bilirubin, the diazo reagent on the reagent pads 80 undergoes a color reaction with bilirubin, as explained above. The color reaction corresponds to the bilirubin concentration. The abovementioned reflectance R is worked out, for instance, according to Expression 1.

$$R=(r1/r2)/(r3/r4)... \quad \text{Expression 1}$$

r1: reflected light intensity, at the 565 nm wavelength, of the reagent pad portion for bilirubin detection
r2: reflected light intensity, at the 760 nm wavelength, of the reagent pad portion for bilirubin detection
r3: reflected light intensity, at the 565 nm wavelength, of the reagent pad portion for reference
r4: reflected light intensity, at the 760 nm wavelength, of the reagent pad portion for reference

After the reflectance R is worked out, the control unit 6 performs a first determination of whether bilirubin is either positive or negative, on the basis of the reflectance R and data of a predefined calibration curve stored in the storage unit 60 (S5). In the case of a positive, preferably, a ranking such as positive (1+), (2+), (3+) is established in accordance with the bilirubin concentration.

If in the control unit 6 the result of the first determination is bilirubin positive (S6: YES), a second determination is carried out on whether or not the positive is a false positive or the likelihood thereof is high. In the second determination it is determined whether or not the following Expression 2 and Expression 3 hold for the absorbances A1, A2 of urine U as worked out in step S3 (S7). The numerical values in Expression 2 and Expression 3 are numerical values per 10 mm of optical path length; herein the absorbance can be appropriately modified in accordance with the measured or converted optical path length.

$$A1<0.08... \quad \text{Expression 2}$$

$$A2<0.5... \quad \text{Expression 3}$$

If both Expression 2 and Expression 3 hold (S7: YES), the control unit 6 determines a false positive or a high likelihood thereof (S8).

Validation results such as the following are obtained when performing re-testing in accordance with a method (for instance, Ictotest) that is different from the above-described analysis device AN, for multiple urine cases in which the result of the first determination is bilirubin positive. Even if the result of the first determination for urine U of transparent- or yellow-grade color tone is bilirubin positive, re-testing of the foregoing results in a high frequency of negatives (false positives), among which cases of urine U having a predefined transparent- or yellow-grade color tone are all negative (false positives). Expression 2 and Expression 3 are expressions for determining whether the color tone of the urine U is a predefined transparent- or yellow-grade color tone. The color tone of the urine U can be determined to be a predefined transparent- or yellow-grade color tone if both Expression 2 and Expression 3 hold. Therefore, it becomes possible to ultimately determine a bilirubin false positive or a high likelihood thereof, if the result of the first determination is bilirubin positive and Expression 2 and Expression 3 hold. The reliability of the bilirubin testing result by the above-described analysis method can be accordingly increased.

The inventors validated the above-described analysis method by carrying out the latter using an analysis device of structure identical to that of the analysis device AN. Thereupon, it was possible to determine, as a result of a second determination, that a total of 69 samples were false positives from among a total of 85 samples of false positives that yielded bilirubin positive in the first determination and that were deemed to be negative through re-testing by Ictotest. This indicates that the above-described analysis method allows increasing the reliability of the analysis results. This increased reliability of analysis is advantageous in terms of incurring fewer re-tests of urine, and affording improved efficiency in the analysis process.

If, unlike in the above-described instances, the result of the first determination is bilirubin negative (S6: NO), or if Expression 2 and Expression 3 do not hold, even if the result of the first determination is bilirubin positive (S7: NO), then the control unit 6 determines whether or not abnormal coloration has occurred in the reagent pads 80 spotted with the urine U (S10). This determination is performed in accordance with Expression 4 below. In Expression 4, R' denotes the light reflectance of spotting sites of urine U, and is worked out on the basis of Expression 5.

$$R'>1.30... \quad \text{Expression 4}$$

$$R' = (r1/r5)/(r3/r6) \ldots \text{ Expression 5}$$

r5: reflected light intensity, at the 500 nm wavelength, of the reagent pad portion for bilirubin detection

r6: reflected light intensity, at the 500 nm wavelength, of the reagent pad portion for reference

r1, r3: identical to r1 and r3 of Expression 1.

If Expression 4 holds, the control unit 6 determines that abnormal coloration has occurred (S10: YES). If abnormal coloration occurs, there is a high likelihood that the result of the previous first determination is inaccurate.

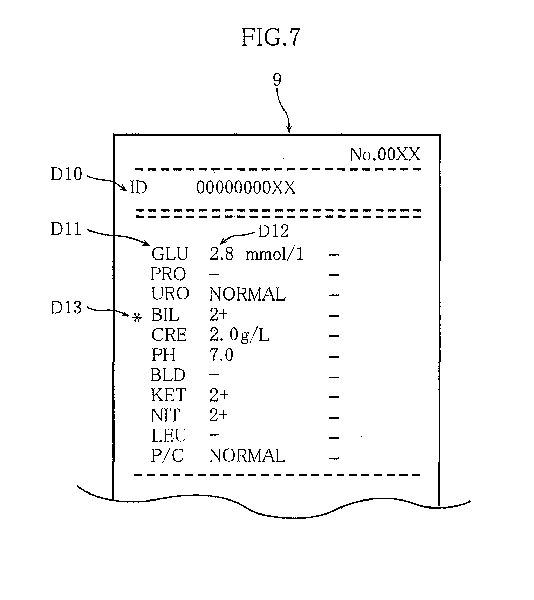

Once a series of processes such as the above-described one is over, the control unit 6 causes that determination result to be printed, using the printer 11, on predefined paper (S9). If the second determination is made in addition to the first determination, that fact is also printed using a symbol or the like that for identifying that occurrence. A report 9, for instance such as the one illustrated in Fig. 7, is created as a result. The printed output of the report 9 includes data D10 for subject identification, data D11 on testing items, and data D12 on testing results. If it is determined that bilirubin is a false positive or that the likelihood of a false positive is high, data D13 that denotes that fact is printed out for the bilirubin (BIL) item. In the figures, data D13 is displayed in the form of a symbol "*", but other symbols, characters or designs can be used instead.

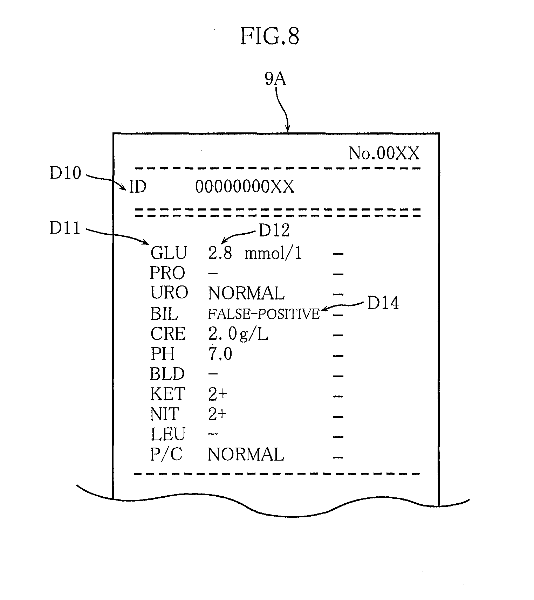

Fig. 8 illustrates an example of another report that is printed by the printer 11 of the analysis device AN. In the figure the reference numerals relating to data are identical to those for data in the report 9 explained for Fig. 7, and will not be explained again. If in a report 9A it is determined that bilirubin is a false positive or that the likelihood of false positive is high, data D14 that denotes that fact is printed out, instead of the testing result data, in the item column of the bilirubin testing result. In the figure, the data D14 is displayed using the characters "FALSE-POSITIVE", but other symbols, characters or designs can be used instead. Needless to say, data that denotes a bilirubin false positive, or a high likelihood thereof, may be set forth alongside the testing result data in the item column on bilirubin testing results.

Through the creation of such reports 9, 9A it becomes possible for the testing personnel using the analysis device AN to perceive, quickly and easily, that bilirubin is a false positive or that the likelihood thereof is high. Printing to the effect that bilirubin is a false positive may be performed in forms that differ from those of the above-described reports 9, 9A

Although not illustrated in the drawings, if in step S10 abnormal coloration is determined to have occurred, information denoting that fact is printed for the item on bilirubin in the report 9. As a result, the testing personnel can accurately perceive that the testing results on bilirubin are suspicious, and that re-testing is necessary. Needless to say, the information takes the form of symbols, characters and designs that can be distinguished from information that denotes a false positive.

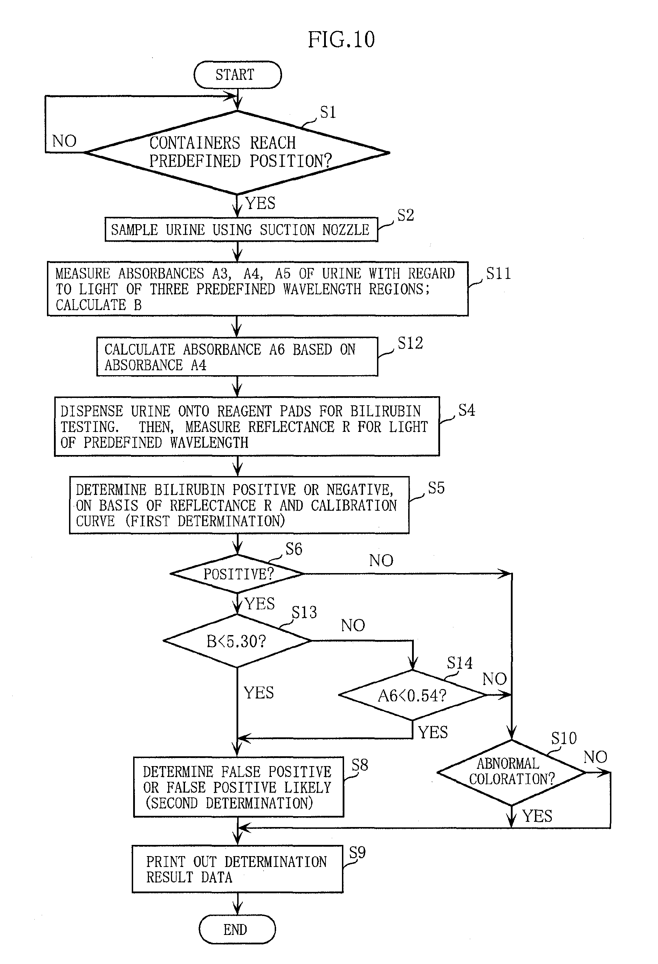

An explanation follows next, with reference to the flowchart illustrated in Fig. 10, on another example of an analysis method of urine U that utilizes the analysis device AN. An example of an instance of the analysis method in which positive and negative bilirubin in the urine U are tested will be explained as well. Another example of the operation processing procedure in the control unit 6 will be explained in conjunction therewith.

In Fig. 10, processes identical or similar to those of the steps in Fig. 6 are carried out in steps denoted by the same numerals as in Fig. 6. Differences between Fig. 10 and Fig. 6 include the following features. Specifically, step S3 and S7 of Fig. 6 have been eliminated in Fig. 10, and step S11, S12, S13 and S14 have been added. The following explanation focus on these additional steps and related steps. A detailed explanation of other steps will be omitted.

When the containers 30 reach a predefined position (S1: YES), the control unit 6 drives the dispensing device 5, and urine U is sampled out of the containers 30 by the suction nozzle 50 (S2). Some of the sampled urine U is infused into the cell 75 of the optical measurement unit for urine tone testing 7B. The control unit 6 causes light to be irradiated, towards the urine U, out of the light source 77 of the optical measurement unit 7B, and absorbances A3, A4, A5 of the urine U with regard to light of the three wavelength regions is worked out from the amount of transmitted light. The control unit 6 calculates a calculated value B on the basis of Expression 6, using the absorbances A3, A4, A5 (S11).

$$B = (A4-A5)/(A3-A5) \ldots \text{ Expression 6}$$

The absorbance A3 is the absorbance of the urine U with regard to light of, for instance, a wavelength of 525 nm or about 525 nm. The absorbance A4 is the absorbance of the urine U with regard to light of, for instance, a wavelength of 470 nm or about 470 nm. The absorbance A5 is the absorbance of the urine U with regard to light of, for instance, a wavelength of 635 nm or about 635 nm. In a case where, for instance, the inner diameter of the cell 75 illustrated in Fig. 5 is 1.8 mm, the absorbances A3, A4, A5 are measured as absorbance for an optical path length of 1.8 mm. However, the absorbances A3, A4, A5 may be converted to absorbances per other optical path length, for instance 5 mm, 10 mm, 15 mm and 20 mm. For instance, an absorbance converted to absorbance per 10 mm of optical path length can be worked out by multiplying the optical path length of 1.8 mm, obtained using the cell 75, by the value (10/1.8). The calculated value B as calculated from the absorbances A3, A4, A5 is used for determining a bilirubin false positive, as described below.

As pointed out above, the measurement wavelength of the absorbance A3 is, for instance, a wavelength of 525 nm or about 525 nm. As Fig. 9 shows, the measurement wavelength of the absorbance A3 matches the wavelength at the foot portion of the absorption of bilirubin. Specifically, the measurement wavelength of the absorbance A3 is preferably selected from 515 nm to 575 nm (wavelength region W1), as in the case of the above-described absorbance A1. The measurement wavelength of the absorbance A4 is, for instance, 470 nm or about 470 nm. The measurement wavelength of the absorbance A4 matches the wavelength at a main absorption portion of bilirubin. Specifically, the measurement wavelength of the absorbance A4 is preferably selected from 400 nm to 480 nm (wavelength region W2), as in the case of the abovementioned A2. The measurement wavelength of the absorbance A5 is, for instance, a wavelength of 635 nm or about 635 nm. The measurement wavelength of the absorbance A5 matches the wavelength at a portion of no absorption of bilirubin. Specifically, the measurement wavelength of the absorbance A5 is preferably selected from 625 nm to 780 nm (wavelength region W3).

Next, absorbance A6 is calculated on the basis of the absorbance A4 (S12). The absorbance A6 corresponds to an example of data of light absorption characteristics in the present invention. The absorbance A6 is the absorbance of the urine with respect to light of wavelength 470 nm or about 470 nm, as absorbance per 10 mm of optical path length. The absorbance A6 results from conversion, to absorbance per 10 mm of optical path length, of the absorbance A4 of the urine U with regard to the above-described light of wavelength 470 nm or about 470 nm. If the absorbance A4 has been converted to absorbance per 10 mm of optical path length, the value of the absorbance A4 can be used, as-is, as the absorbance A6. Needless to say, the absorbance A6 may be converted to absorbances per other optical path length, for instance 5 mm, 15 mm and 20 mm. The absorbance A6 may be measured separately from the absorbance A4.

Next, the urine U sampled by the suction nozzle 50 is spotted onto the reagent pads 80 for bilirubin testing of the specimens 8, in the same way as explained for the flowchart of Fig. 6. Thereafter, light of two wavelengths is sequentially irradiated onto the spotting portions, to measure thereby the reflectance R of light from the spotting portions (S4). After the reflectance R is worked out, the control unit 6 performs a first determination of whether bilirubin is either positive or negative, on the basis of the reflectance R and data of a predefined calibration curve stored in the storage unit 60 (S5).

If the result of the first determination is bilirubin positive (S6: YES), the control unit 6 carries out a second determination of whether or not the positive is a false positive or the likelihood thereof is high. In the second determination it is determined whether Expression 7 holds for the calculated value B worked out in step S11 (S13).

$$B < 5.30 \ldots \quad \text{Expression 7}$$

If Expression 7 holds (13: YES), the control unit 6 determines a false positive or a high likelihood thereof (S8).

If Expression 7 does not hold (S13: NO), the control unit 6 performs an additional determination of whether or not Expression 8 holds (S14) with regard to the absorbance A6. The numerical value in Expression 8 is a numerical value per 10 mm of optical path length. The absorbance can be appropriately modified in accordance with the measured or converted optical path length.

$$A6 < 0.54 \ldots \quad \text{Expression 8}$$

If Expression 8 holds (S14: YES), the control unit 6 determines, in the second determination, that there is a false positive or a high likelihood thereof (S8).

The purpose of Expression 7 is to discriminate precisely false-positive urine from urine for which the result of the first determination is bilirubin positive. As described above, cases of urine having a predefined transparent- or yellow-grade color tone are all negative (false positives). Like Expression 2 and Expression 3, Expression 7 is an expression for determining whether the color tone of the urine U is a predefined transparent- or yellow-grade color tone. The color tone of the urine U can be determined to be a predefined transparent- or yellow-grade color tone if Expression 7 holds. Therefore, it becomes possible to ultimately determine a bilirubin false positive or a high likelihood thereof, if the result of the first determination is bilirubin positive and Expression 7 holds. The reliability of the bilirubin testing result by the above-described analysis method can be increased thereby.

Expression 8 is an expression for additionally determining whether or not there is a false positive, in case that Expression 7 does not hold. If Expression 8 holds, it can be ultimately determined that there is a bilirubin false positive or a high likelihood thereof, even for urine U such that the result of the first determination is bilirubin positive but Expression 7 does not hold. The reliability of the bilirubin testing result by the above-described analysis method can be increased thereby.

The inventors collected urine U of transparent- or yellow-grade color tone, and validated the above-described analysis method by carrying out the latter using an analysis device of structure identical to that of the analysis device AN. Thereupon, through determination using Expression 7, it was possible to determine, as a result of a second determination, that a total of 87 samples were false positives, from among a total of 88 samples of false positives that yielded bilirubin positive as the result of the first determination, and that were deemed to be negative through re-testing by Ictotest. Through additional determination using Expression 8, it was possible to determine, as a result of a second determination, that a total 88 samples were false positives from among the total of 88 samples of the abovementioned false positives.

Determination based on Expression 7 and Expression 8 was performed on 7 samples of transparent- or yellow-grade color tone for which the result of the first determination was bilirubin positive and that yielded positive also according to re-testing by Ictotest. No sample was determined to be a bilirubin false positive or to have a high likelihood thereof.

This indicates that the above-described analysis method allows increasing the reliability of the analysis results. This increased reliability of analysis is advantageous in terms of incurring fewer re-tests of urine, and affording improved efficiency in the analysis process.

If the result of the first determination is bilirubin negative (S6: NO), or if Expression 8 does not hold even if the result of the first determination is bilirubin positive (S14: NO), the control unit 6 determines whether or not abnormal coloration has occurred in the reagent pads 80 spotted with the urine U, in the same way as explained for the flowchart illustrated in Fig. 6 (S10).

Once the series of processes such as the above-described ones is over, the control unit 6 causes that determination result to be printed, using the printer 11, on predefined paper in the same way as in explained for the flowchart of Fig. 6 (S9).

The present invention is not limited to the features of the above-described embodiments.

Expressions other than Expression 2, Expression 3, Expression 7 and Expression 8 can be used as the condition for determining whether the bilirubin positive is a false positive. A simple value of light transmittance may be used, instead of absorbance, as the "data on light absorption characteristics of the urine with regard to light of a predefined wavelength region" according to the present invention. Light reflectance can likewise be used, since it also denotes light absorption characteristics. Instances where a first determination result of bilirubin positive is ultimately deemed to be a false positive are fundamentally instances where a color tone of urine is a predefined colorless- or yellow-grade color tone. Therefore, a procedure can be used that involves, for instance, irradiating urine with light of a wide wavelength region, performing a spectral analysis of the transmitted light, determining as a result the color tone of the urine, and, on the basis of that determination result, determining whether or not there is a false positive.

In the above-described embodiments a typical example has been explained relating to bilirubin testing, but the present invention can be used also for testing items other than bilirubin (for instance, urobilinogen or the like). The present invention is not limited to determination of false positives, and can be used for determining also false negatives. The present invention can be used not only in comparatively large analysis devices provided with a transport device for containers, but also in small analysis devices that lack such a transport device.

In the above-described embodiments, the optical measurement unit for urine tone testing 7B provided in the analysis device AN is used for measuring the absorbance of urine, but there may be provided a separate optical measurement unit for measuring the absorbance that is used for determining false positives and false negatives.

In the above-described embodiments, "data on light absorption characteristics of the urine with regard to light of a predefined wavelength region" is acquired using light of wavelength regions in the vicinity of 525 nm, 470 nm and 635 nm, but light of other wavelength regions may be used instead.

EXPLANATION OF REFERENCE NUMERALS

AN: analysis device
U: urine
6: control unit (determination means)
7A: optical measurement unit for color reaction sensing
7B: optical measurement unit for urine tone testing

(optical measurement means)

8: specimen
11: printer (data output means)
12: display unit (data output means)
80: reagent pad

**Claims**

1. A urine analysis method, comprising:

   a first determination step of, on the basis of a color reaction between a reagent and a specific component in urine, determining a distinction between a positivity and negativity of the specific component; and
   a step of working out data on light absorption characteristics of the urine itself with respect to light of a predefined wavelength region,
   this method further comprising:

   a second determination step of, when a value of the data on the light absorption characteristics lies within a predefined range and a determination result in the first determination step is either positive or negative as established beforehand, changing the determination result to a false positive or false negative, or determining that there is a likelihood of a false positive or a false negative.

2. The urine analysis method according to claim 1, wherein in the second determination step the value of the data on the light absorption characteristics is determined to lie within the predefined range when the urine is of a predefined colorless grade or yellow grade.

3. The urine analysis method according to claim 1 or 2, wherein
   the specific component in the urine is bilirubin, and
   when the value of the data on the light absorption characteristics lies within the predefined range and the determination in the first determination step has turned out to be bilirubin positive, in the second determination step the positive is changed to a false positive, or determination is made that there is a likelihood of a false positive.

4. The urine analysis method according to claim 3,
   wherein the data on the light absorption characteristics is data denoting light absorption characteristics of the urine with respect to light of a wavelength in the vicinity of 525 nm and of a wavelength in the vicinity of 470 nm.

5. The urine analysis method according to claim 4, wherein
   determination is made, in the second determination step, that the value of the data on the light absorption characteristics lies within the predefined range
   when there hold both relationships in expressions:

$$A1 < 0.08$$

$$A2 < 0.5$$

   where A1 denotes absorbance of the urine with respect to light of wavelength in the vicinity of 525 nm and absorbance per 10 mm of optical path length, and
   A2 denotes absorbance of the urine with respect to light of wavelength in the vicinity of 470 nm and absorbance per 10 mm of optical path length.

6. The urine analysis method according to claim 3,
   wherein the data on the light absorption characteristics is a calculated value B that is calculated according to expression:

$$B=(A4-A5)/(A3-A5)$$

(where A3, A4 and A5 are, respectively, the absorbance of the urine with respect to light of a wavelength in the vicinity of 525 nm, a wavelength in the vicinity of 470 nm and of a wavelength in the vicinity of 635 nm.)

7. The urine analysis method according to claim 6, wherein determination is made whether a relationship B<5.30 holds or not.

8. The urine analysis method according to claim 7, wherein when the relationship B<5.30 holds, determination is made, in the second determination step, that the value of the data on the light absorption characteristics lies within the predefined range.

9. The urine analysis method according to claim 7, wherein
when the relationship B<5.30 does not hold, further determination is made as to whether a relationship A6<0.54 holds or not, and when the relationship A6<0.54 holds, determination is made, in the second determination step, that the value of the data on the light absorption characteristics lies within the predefined range
(where A6 denotes absorbance of the urine with respect to light of wavelength in the vicinity of 470 nm and absorbance per 10 mm of optical path length).

10. The urine analysis method according to any of claims 1 to 9, further comprising a step of, when predefined abnormal coloration occurs in the color reaction, detecting that occurrence.

11. An analysis device, comprising:

determination means capable of, on the basis of a color reaction between a reagent and a specific component in urine, performing a first determination of distinguishing between a positivity and negativity of the specific component, and
optical measurement means capable of working out data on light absorption characteristics of the urine itself with respect to light of a predefined wavelength region,
wherein the determination means is configured so that, when a value of the data on the light absorption characteristics lies within a predefined range and a determination result in the first determination is either positive or negative as established beforehand, the determination means changes the result of the first determination to a false positive or false negative, or performs a second determination to the effect that there is a likelihood of a false positive or a false negative.

12. The analysis device according to claim 11, wherein in the second determination the value of the data on the light absorption characteristics is determined to lie within the predefined range when the urine is of a predefined colorless grade or yellow grade.

13. The analysis device according to claim 11 or 12,
wherein the determination means is configured so that, in case of determining a distinction between a positivity and negativity of bilirubin as the specific component in the urine, and
when the value of the data on the light absorption characteristics lies within the predefined range and a result of the first determination is bilirubin positive, the determination means, in the second determination, changes the positive to a false positive or determines that there is a likelihood of a false positive.

14. The analysis device according to claim 13, wherein the data on the light absorption characteristics is data denoting light absorption characteristics of the urine with respect to light of a wavelength in the vicinity of 525 nm and of a wavelength in the vicinity of 470 nm.

15. The analysis device according to claim 14, wherein determination is made, in the second determination, that the value of the data on the light absorption characteristics lies within the predefined range, when there hold both relationships in expressions:

$$A1 < 0.08$$

$$A2 < 0.5$$

where A1 denotes absorbance of the urine with respect to light of wavelength in the vicinity of 525 nm and absorbance per 10 mm of optical path length, and

A2 denotes absorbance of the urine with respect to light of wavelength in the vicinity of 470 nm and absorbance per 10 mm of optical path length.

16. The analysis device according to claim 13, wherein the data on the light absorption characteristics is a calculated value B that is calculated according to expression:

$$B = (A4 - A5) / (A3 - A5)$$

(where A3, A4 and A5 are, respectively, the absorbance of the urine with respect to light of a wavelength in the vicinity of 525 nm, a wavelength in the vicinity of 470 nm and of a wavelength in the vicinity of 635 nm.)

17. The analysis device according to claim 16, configured so as to determine whether B<5.30 holds or not.

18. The analysis device according to claim 17, wherein when the relationship B<5.30 holds, determination is made, in the second determination, that the value of the data on the light absorption characteristics lies within the predefined range.

19. The analysis device according to claim 17, wherein when the relationship B<5.30 does not hold, further determination is made as to whether a relationship A6<0.54 holds or not, and when the relationship A6<0.54 holds, determination is made, in the second determination, that the value of the data on the light absorption characteristics lies within the predefined range (where A6 denotes absorbance of the urine with respect to light of wavelength in the vicinity of 470 nm and absorbance per 10 mm of optical path length).

20. The analysis device according to any of claims 11 to 19, wherein data for identifying the result of the second determination can be outputted using data output means.

21. The analysis device according to any of claims 11 to 20, configured in such a manner that when predefined abnormal coloration occurs in the color reaction, that occurrence is detected.

22. A program for causing determination means of an analysis device to perform data processing, the analysis device being provided with:

determination means capable of, on the basis of a color reaction between a reagent and a specific component in urine, performing a first determination of distinguishing between a positivity and negativity of the specific component, and

optical measurement means capable of working out data on light absorption characteristics of the urine itself with respect to light of a predefined wavelength region,

wherein the program comprises data for causing the determination means to execute a second determination of, when a value of the data on the light absorption characteristics lies within a predefined range and a determination result in the first determination step is either positive or negative as established beforehand, changing the determination result to a false positive or false negative, or determining that there is a likelihood of a false positive or a false negative.

23. A storage medium, which stores the program according to claim 22.

FIG.1

EP 2 579 035 A1

FIG.2

FIG.3

EP 2 579 035 A1

FIG.4

FIG.5

## FIG.6

```
                    ( START )
                        │
        ┌───────────────┤
        │               ▼                        S1
      NO    ◇ CONTAINERS REACH ◇
             ◇ PREDEFINED POSITION? ◇
                        │
                        │ YES
                        ▼                         S2
        ┌──────────────────────────────────┐
        │ SAMPLE URINE USING SUCTION NOZZLE │
        └──────────────────────────────────┘
                        │
                        ▼                         S3
        ┌──────────────────────────────────┐
        │ MEASURE ABSORBANCES A1, A2 OF     │
        │ URINE WITH REGARD TO LIGHT OF TWO │
        │ PREDEFINED WAVELENGTH REGIONS     │
        └──────────────────────────────────┘
                        │
                        ▼                         S4
        ┌──────────────────────────────────────┐
        │ DISPENSE URINE ONTO REAGENT PADS FOR  │
        │ BILIRUBIN TESTING.                    │
        │ THEN, MEASURE REFLECTANCE R FOR LIGHT │
        │ OF PREDEFINED WAVELENGTH              │
        └──────────────────────────────────────┘
                        │
                        ▼                         S5
        ┌──────────────────────────────────────┐
        │ DETERMINE BILIRUBIN POSITIVE OR       │
        │ NEGATIVE, ON BASIS OF REFLECTANCE R   │
        │ AND CALIBRATION CURVE (FIRST          │
        │ DETERMINATION)                        │
        └──────────────────────────────────────┘
                        │        S6
                        ▼            NO
                  ◇ POSITIVE? ◇ ──────────────────┐
                        │                          │
                        │ YES                      │
                        ▼          S7              │
                  ◇ A1<0.08?  ◇      NO            ▼         S10
                  ◇   and     ◇ ─────────►  ◇ ABNORMAL   ◇  NO
                  ◇ A2<0.5?   ◇             ◇ COLORATION? ◇ ───┐
                        │                          │           │
                        │ YES     S8               │ YES       │
                        ▼                          ▼           │
        ┌──────────────────────────┐               │           │
        │ DETERMINE FALSE POSITIVE  │               │           │
        │ OR FALSE POSITIVE LIKELY  │               │           │
        │ (SECOND DETERMINATION)    │               │           │
        └──────────────────────────┘               │           │
                        │◄──────────────────────────┘           │
                        ▼          S9                            │
        ┌──────────────────────────┐                            │
        │ PRINT OUT DETERMINATION   │◄───────────────────────────┘
        │ RESULT DATA               │
        └──────────────────────────┘
                        │
                        ▼
                    ( END )
```

FIG.7

9

|         |            | No.00XX |
|---------|------------|---------|
| D10 → ID | 00000000XX |         |

D11 ─→                    ┌─D12
      GLU    2.8 mmol/1    –
      PRO    –             –
      URO    NORMAL        –
D13 ─→ * BIL   2+          –
      CRE    2.0g/L        –
      PH     7.0           –
      BLD    –             –
      KET    2+            –
      NIT    2+            –
      LEU    –             –
      P/C    NORMAL        –

FIG.8

9A

No.00XX

D10 —→ ID    00000000XX

D11 —→
      D12
      GLU   2.8 mmol/1    –
      PRO   –             –
      URO   NORMAL        –
      BIL   FALSE-POSITIVE –  D14
      CRE   2. 0g/L        –
      PH    7.0           –
      BLD   –             –
      KET   2+            –
      NIT   2+            –
      LEU   –             –
      P/C   NORMAL        –

FIG.9

FIG.10

```
                    ┌─────────────┐
                    │   START     │
                    └──────┬──────┘
                           │
        ┌──────────────────┤
        │                  ▼
        │           ╱‾‾‾‾‾‾‾‾‾‾╲  S1
     NO │        ╱ CONTAINERS REACH ╲
        └───────┤ PREDEFINED POSITION? │
                 ╲                    ╱
                    ╲‾‾‾‾‾‾‾‾‾‾‾‾‾‾╱
                           │ YES
                           ▼
              ┌────────────────────────────────┐  S2
              │ SAMPLE URINE USING SUCTION NOZZLE │
              └───────────────┬────────────────┘
```

╱‾‾‾‾‾‾‾‾‾‾‾‾╲ S1
CONTAINERS REACH
PREDEFINED POSITION?

SAMPLE URINE USING SUCTION NOZZLE ⟋S2

MEASURE ABSORBANCES A3, A4, A5 OF URINE WITH REGARD ⟋S11
TO LIGHT OF THREE PREDEFINED WAVELENGTH REGIONS;
CALCULATE B

CALCULATE ABSORBANCE A6 BASED ON ⟋S12
ABSORBANCE A4

DISPENSE URINE ONTO REAGENT PADS FOR BILIRUBIN ⟋S4
TESTING. THEN, MEASURE REFLECTANCE R FOR LIGHT
OF PREDEFINED WAVELENGTH

DETERMINE BILIRUBIN POSITIVE OR NEGATIVE, ⟋S5
ON BASIS OF REFLECTANCE R AND CALIBRATION
CURVE (FIRST DETERMINATION)

⟋S6
POSITIVE? —— NO ——→

↓ YES ⟋S13    NO
B<5.30? ————————→

⟋S14    NO
A6<0.54? ————————→

YES        YES

⟋S10    NO
ABNORMAL ————————→
COLORATION?

↓ YES

DETERMINE FALSE POSITIVE ⟋S8
OR FALSE POSITIVE LIKELY
(SECOND DETERMINATION)

PRINT OUT DETERMINATION ⟋S9
RESULT DATA

┌─────────────┐
│    END      │
└─────────────┘

| INTERNATIONAL SEARCH REPORT | International application No. |
|---|---|
| | PCT/JP2011/061753 |

A. CLASSIFICATION OF SUBJECT MATTER
*G01N33/493*(2006.01)i, *G01N21/78*(2006.01)i, *G01N33/483*(2006.01)i

According to International Patent Classification (IPC) or to both national classification and IPC

B. FIELDS SEARCHED

Minimum documentation searched (classification system followed by classification symbols)
G01N33/493, G01N21/78, G01N33/483

Documentation searched other than minimum documentation to the extent that such documents are included in the fields searched
Jitsuyo Shinan Koho 1922-1996 Jitsuyo Shinan Toroku Koho 1996-2011
Kokai Jitsuyo Shinan Koho 1971-2011 Toroku Jitsuyo Shinan Koho 1994-2011

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | Naoko KITAMURA et al., "Zenjido Nyo Bunseki Sochi Aution Max AX-4030 no Seino Hyoka", The Journal of Clinical Laboratory Instruments and Reagents, 10 October 2008 (10.10.2008), vol.31, no.5, pages 583 to 594 | 1-23 |
| A | Kaori HIRAYAMA et al., "Sokutei Kiki no Shikicho Hantei o Mochiita Nyo Teiseiho no Check Logic", Shiritsu Ika Daigaku Rinsho Kensa Gishi Kaishi, 30 September 2009 (30.09.2009), no.49, page 10 | 1-23 |
| A | Yoshio ARAI et al., "Automatic determination of urine color tone by an autoanalyser", The Journal of Clinical Laboratory Instruments and Reagents, 10 June 1995 (10.06.1995), vol.18, no.3, pages 567 to 574 | 1-23 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03 August, 2011 (03.08.11) | 16 August, 2011 (16.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/061753 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 63-142264 A  (Nittec Co., Ltd.),<br>14 June 1988 (14.06.1988),<br>(Family: none) | 1-23 |
| A | JP 02-161354 A  (Secom Co., Ltd.),<br>21 June 1990 (21.06.1990),<br>(Family: none) | 1-23 |
| A | JP 07-035744 A  (Fujisawa Pharmaceutical Co.,<br>Ltd.),<br>07 February 1995 (07.02.1995),<br>(Family: none) | 1-23 |
| A | JP 07-301630 A  (Kyoto Daiichi Kagaku Co.,<br>Ltd.),<br>14 November 1995 (14.11.1995),<br>& US 5772606 A          & EP 670492 A2<br>& DE 69520850 D          & CN 1118068 A | 1-23 |
| A | JP 10-048207 A  (Bayer Corp.),<br>20 February 1998 (20.02.1998),<br>& US 5724148 A          & EP 806662 A2<br>& DE 69731327 D          & AU 2012397 A<br>& CA 2204305 A          & ES 2231831 T | 1-23 |
| A | Haruo AMANO et al., "'US-3100Rplus' ni Okeru<br>Chakushokunyo Oyobi Nyo Hiju no Kento", Igaku<br>Kensa, 25 April 2010 (25.04.2010), vol.59,<br>no.4, page 516 | 1-23 |
| A | Toshiyuki AKIYAMA et al., "Basic examination<br>on automatic urine inspection apparatus<br>'Urocheck'", The Journal of Clinical Laboratory<br>Instruments and Reagents, 10 February 1995<br>(10.02.1995), vol.18, no.1, pages 43 to 52 | 1-23 |
| P,X/P,A | Kaori HIRAYAMA et al., "AX-4030 no Nyo Shikicho<br>Hantei o Riyo shite Bilirubin Teisei Giyosei<br>Kentai o Kenshutsu Dekiruka", Japanese Journal<br>of Clinical Laboratory Automation, 01 September<br>2010 (01.09.2010), vol.35, no.4, page 715 | 1-3/4-23 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H735744 B
- JP 2009229233 A

- JP 2000321270 A